# EUROPEAN PATENT APPLICATION

(11) **EP 1 902 671 A1**
(43) Date of publication of application: **26.03.2008**
(21) Application number: 07117022.9
(22) Date of filing: 24.09.2007
(51) Int. Cl.: A61B 3/00, A61B 18/22

(54) **Eye surgical illumination unit, light guide, method, computer program product and computer system.**

(30) Priority: 22.09.2006 NL 1032559
(71) Applicant: D.O.R.C. Dutch Ophthalmic Research Center (International) B.V., 3214 VN Zuidland (NL)
(72) Inventor: Vijfvinkel, Gerrit Jan, 3211 BA, Geervliet (NL)
(74) Representative: Hatzmann, Martin

(57) **Abstract**

The invention relates to an eye surgical illumination unit, comprising a light source for visual inspection of eye tissue to be treated, a connector for light guides for guiding light exiting from the light source to the eye tissue, and optical elements for defining a light path along which light exiting from the light source can travel to the connector, and a housing in which the light source and the optical elements are accommodated. Further, the illumination unit is arranged for an improved tuning of light operatively traveling along the light path, to a light guide connectible to the connector.

## Description

This invention relates to an eye surgical illumination unit, comprising a light source for visual inspection of eye tissue to be treated, a connector for light guides for guiding light exiting from the light source to the eye tissue, and optical elements for defining a light path along which light exiting from the light source can travel to the connector, and a housing in which the light source and the optical elements are accommodated.

Such an eye surgical illumination unit is for instance known from US 5 658 070, in which a unit is described with two connectors for light guides. The optical elements comprise reflectors and lenses for defining the light path to the connectors. The light source arranged in the housing, designed as a bulb, operatively generates light which is guided via the optical elements along the light paths to the connectors for coupling into connected light guides.

Use of such an illumination unit, however, requires the necessary experience. For instance, the material of specific light guides can degenerate irreversibly when the light intensity of the illumination unit has been set too high. On the other hand, too low a light intensity may lead to poor *in situ* illumination, which renders the surgical procedures to be performed more difficult. Thus, in case of moderate use of documentation material, the utility of the illumination unit is non-optimal.

The invention contemplates an eye surgical illumination unit of the type described in the opening paragraph hereof, in which, while maintaining the advantages, the disadvantages mentioned are obviated. In particular, the invention contemplates obtaining an eye surgical illumination unit that yields increased utility, also when documentation material is consulted to a limited extent only or not consulted at all. To that end, the illumination unit is arranged for an improved tuning of light operatively traveling along the light path, to a light guide connectible to the connector.

The invention is based on the insight that realizing an improved tuning of light operatively traveling along the light path, to a light guide connectible to the connector leads to fewer unwanted illumination situations. Thus, the chance of either too high or too low a light yield in the light guide can decrease. Consequently, the utility of the illumination unit increases without a user needing to examine documentation material of the illumination unit in depth. Thus, users can focus more on primary activities in eye surgical procedures.

It is noted that a light guide is understood to mean a structure for guiding light, such as an optical fiber.

Preferably, the illumination unit is arranged for generating information based on a measurement on a light guide connectible to the connector, for direct tuning of light operatively exiting from the illumination unit via the connector. In this way, based on measured physical quantities, without intervention of for instance conversion tables, a tuning of light operatively exiting from the light source can come about. Thus, relatively easily, an improved tuning of the light to a light guide connectible to the connector can be realized.

In an advantageous embodiment, the illumination unit comprises an optical measuring device for identifying a light guide connectible to the connector. By performing an optical identification, it can be established in a relatively simple and inexpensive manner what type of light guide is being connected to the illumination unit. Thus, information can be provided for direct tuning of the light exiting during use. On the basis of setting characteristics of the identified light guide, the user can then effect, possibly manually, an optimal light tuning. In a user-friendly embodiment, such setting characteristics are stored in a memory element in the illumination unit, so that these characteristics can be shown to the user, preferably automatically, via a display image. In a particularly user-friendly embodiment, the tuning of the light during use is realized automatically in response to the above-mentioned measurement, on the basis of the stored setting characteristics of the identified light guide. Incidentally, it is also possible to tune the light exiting from the illumination unit more generally in response to a measurement on a light guide connectible to the connector, possibly without using stored setting characteristics of the light guide, but using, for instance, codings included in the identification code of the light guide itself, and/or in response to a measurement of a different kind, for instance a mechanical detection.

It is noted that instead of performing a direct optical identification, optionally an optical measurement can be performed on the light guide to find out optical characteristics of the light guide, so that based on such measurements identification of the light guide can take place in an indirect manner.

By the use of an intensity filter comprising a displaceable, substantially optically transparent carrier which is provided with a substantially optically impermeable layer with a coverage varying as a function of the location, in an elegant and well-definable manner the intensity of the light generated by the light source can be tuned to the desired light yield in a connected light guide. The coverage varying as a function of the location, which is preferably locally substantially uniform, defines an intensity transfer by allowing the incident light to pass only partially. By displacing the carrier, the area on which the generated light is incident changes as well, so that as a result of the changed coverage a different intensity transfer is realized. When using a locally substantially uniform coverage, the intensity transfer varies relatively gradually, which is beneficial to the settability of the tuning.

Preferably, the displaceable, substantially optically transparent carrier is designed as a swiveling disc, so that in a compact manner a well-defined displacement of the filter and hence a desired intensity transfer can be realized. Of course, the carrier may also be designed differently, for instance as a slidable, substantially rectangular plate.

By providing the displaceable, substantially optically transparent carrier with an opening for allowing locally unhindered passage of light exiting from the light source, a practically complete transmission of the incident light can be realized, thus allowing the light to sustain no, or substantially no, intensity losses from the filter. Alternatively, it is naturally possible to design an area of the transparent carrier without a substantially optically impermeable layer.

The invention also relates to a light guide for eye surgical applications.

In addition, the invention also relates to a method.

Further, the invention relates to a computer program product.

Furthermore, the invention relates to a computer system.

Further advantageous embodiments of the invention are represented in the subclaims.

The invention will be further elucidated with reference to an exemplary embodiment represented in the drawing. In the drawing:
Fig. 1 shows a schematic perspective view of a cutaway eye surgical illumination unit according to the invention;
Fig. 2A shows a schematic top plan view of the optics of the eye surgical illumination unit of Fig. 1;
Fig. 2B shows a schematic top plan view of a detail of the optics of Fig. 2A;
Fig. 3 shows a schematic front view of an intensity filter from the eye surgical illumination unit of Fig. 1;
Fig. 4 shows a schematic side elevation of an end of a light guide connectible to the eye surgical illumination unit of Fig. 1;
Fig. 5 shows a schematic view of a light beam;
Fig. 6 shows a schematic view of the light source of the illumination unit of Fig. 1; and
Fig. 7 shows a computer system.

The Figures are only a schematic representation of a preferred embodiment of the invention In the figures, equal or corresponding parts are indicated with the same reference numerals.

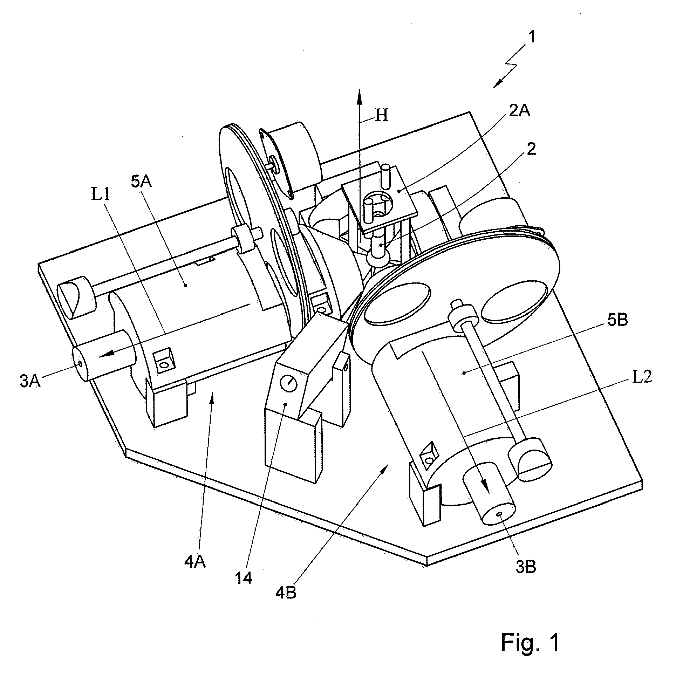

Fig. 1 shows an embodiment of a cutaway eye surgical illumination unit 1 according to the invention in a schematic perspective view. The unit 1 has a light source 2 for visual inspection of eye tissue to be treated, two connectors 3A, 3B for light guides, and optical elements 4A, 4B for defining a light path L1, L2 along which light exiting from the light source can travel to the connectors 3A, 3B. The unit 1 has furthermore a housing (not shown), in which the light source 2 and the optical elements 4A, 4B are accommodated. As will be elucidated hereinafter, the illumination unit 1 is arranged for an improved tuning of light traveling along a light path L1, L2, to a light guide connectible to a connector 3A, 3B. A connected light guide guides light exiting from the illumination unit to the eye tissue.

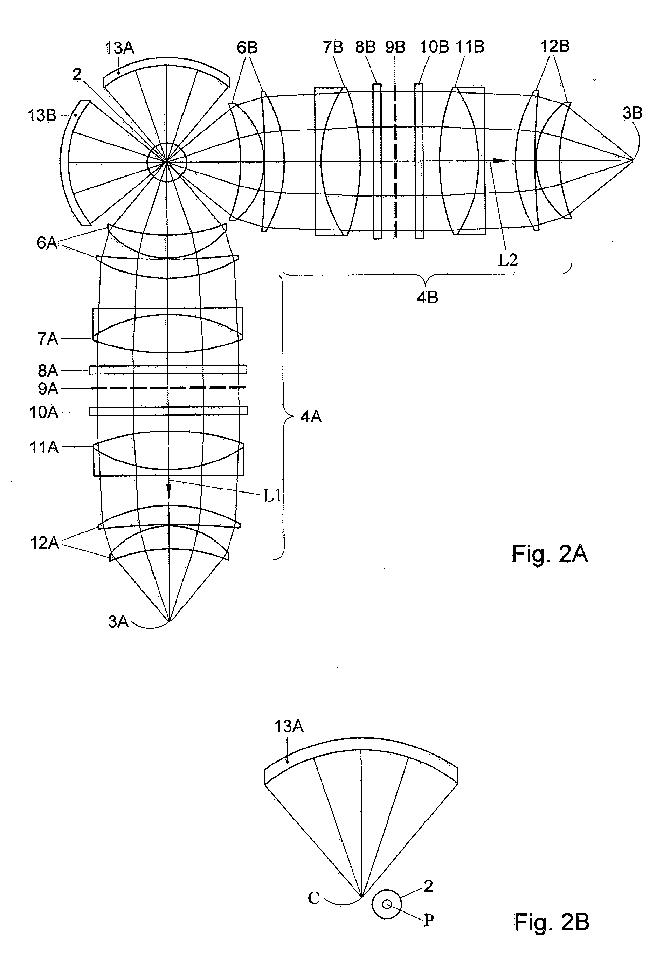

Fig. 2A shows a schematic top plan view of the optics of the eye surgical illumination unit 1. Light exiting from the light source 2 is guided with the aid of the optical elements 4A, 4B along separate light paths L1, L2 to the associated connectors 3A, 3B. The optical elements 4A, 4B comprise per light path L1, L2 a number of individual elements which are accommodated per light path in a cylindrical sleeve 5A, 5B. The individual elements comprise, in the direction of the propagating light, a first set of collimating lenses 6A, 6B, a first individual collimating lens 7A, 7B, a heat mirror 8A, 8B, an intensity filter 9A, 9B, a so-called yellow filter 10A, 10B for absorbing UV radiation, a second individual collimating lens 11A, 11B, and a second set of collimating lenses 12A, 12B. By means of the optical elements 4A, 4B, the light exiting from the light source 2 can be accurately converged, thus allowing efficient coupling into a light guide via the connectors 3A, 3B.

It is noted that the above-described optical elements merely involve an exemplary embodiment. Such a combination of optical elements can naturally be designed differently, for instance without yellow filter or with different numbers of collimating lenses.

Further, it is noted that the numerical aperture (NA) of the optical elements is substantially greater than circa 0.5, for instance in the order of magnitude of 0.6, so that a wider light beam can propagate along the light paths L1, L2. Consequently, the intensity of the light at the connectors 3A, 3B can increase, so that more light becomes available for coupling into light guides. In this way, the intensity of the light can be better tuned to individual light guides. Furthermore, the light exiting from the light source 2 is utilized better, so that, optionally, at a single light source a plurality of separate light paths with associated connectors for light guides may be provided, whereby the light yield, with jointly connected light guides, can simultaneously be at a sufficiently high level. It is also possible to use optical elements that have an NA in the order of magnitude of 0.5, for instance for reducing the cost price of the illumination unit.

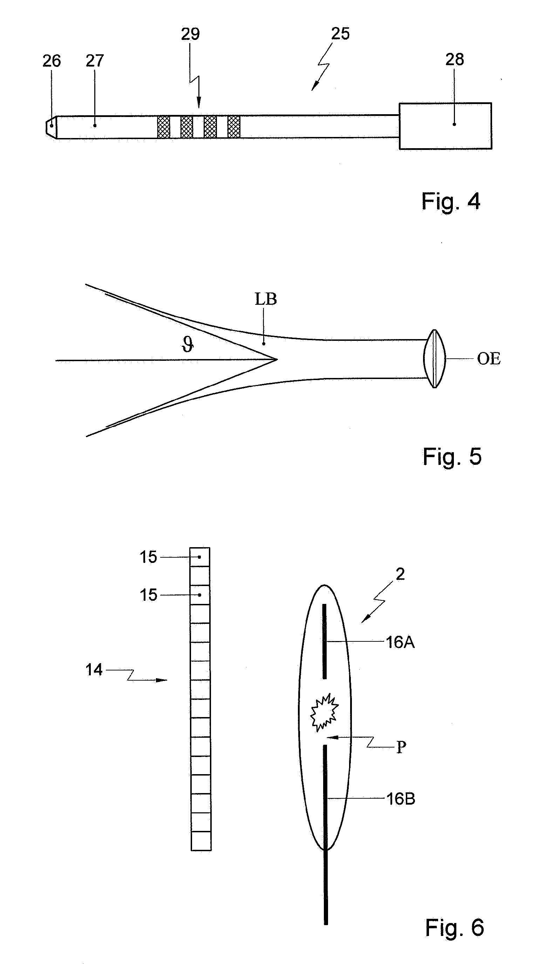

The numerical aperture (NA) of an optical element OE is defined with reference to Fig. 5, which shows a schematic side elevation of a light beam LB which is incident on the optical element OE. The numerical aperture is understood to mean the product of the refractive index of the medium in which the optical element OE has been placed and the sine of the half angle of the maximum cone of light, denoted as θ in Fig. 5, that can penetrate into the optical element OE or can exit from it.

As described above, the illumination unit comprises, apart from a first connector for light guides with associated optical elements for defining a light path along which light exiting from the light source can travel to the first connector, an additional connector with associated optical elements for defining a separate light path. Accordingly, two light guides can be simultaneously provided with light by the illumination unit. In this way, during an eye surgical intervention, two functioning illumination points can be introduced, so that a good illumination is obtained at the site of the intervention. Also, different instruments that are provided with such a light guide can be used alternately without necessitating exchange of light guides or an extra illumination unit. Naturally, the number of connectors with associated separate light paths can vary, being more than two, for instance three, or fewer than two, i.e. just one connector. Use of a multiple number of connectors is advantageous especially where efficient use is made of the light emitted by the light source, for instance by using optical elements with a relatively high numerical aperture.

The optical elements 4A, 4B comprise furthermore per light path L1, L2 a spherical reflector 13A, 13B for reflecting light exiting from the light source 2. The reflected light propagates along the lamp 2, following the respective light paths L1, L2. Thus, a relatively large amount of light is utilized for coupling into the optical light fibers. Especially for use of a multiple number of connectors with associated light paths, it is of importance, as explained above, to utilize the generated light as efficiently as possible. In principle, however, it is also possible to omit the spherical reflectors 13A, 13B, so that costs for optical elements can be saved on. The geometric center C of the reflector 13A has been placed in the heart of the position in which the light is generated, so that an optically optimal geometry is obtained. In Fig. 2B, showing a schematic top plan view of a detail of the optics of Fig. 2A, an alternative configuration is visible. As shown, the geometric center C of the reflector 13A has been displaced to some extent relative to the position P in the light source 2 where during use of the unit 1 the light is generated, for instance the electrodes of an arc lamp. The effect of this is that the amount of reflected light that reaches the position P where during use the light is generated, is limited, which prolongs the service life of the light source 2, while the optical loss of reflected light captured by the optical elements can be relatively minor. Consequently, the utility of the illumination unit 1 can be augmented.

Preferably, a reflector surface of the two spherical reflectors 13A, 13B is provided with an infrared filter. Filtering infrared radiation from the light generated by the light source 2 reduces, through prevention of thermal loading, the chance of damage to the light source 2 proper, as well as to optical structures behind it, such as the optical elements 4A, 4B and a connected light guide. The infrared filter comprises a coating which is reflective to visible light but transparent to infrared light and hence hardly, if at all, reflects radiation having a wavelength of infrared light. Alternatively, the infrared filter can comprise material that absorbs infrared radiation, so that the amount of reflected infrared radiation likewise decreases. Thus, light exiting from the illumination unit 1 is better adjusted to light guides connectible to the unit 1. This is then done not by, or not only by, adjustment of the light intensity, but by adjustment of the spectrum of the generated light. It is noted that the infrared filter can also be arranged on a different optical element, or on a multiple number of them. Also, the use of an infrared filter can naturally be omitted, for instance for reasons of cost.

In a preferred embodiment of the eye surgical illumination unit 1 according to the invention, the infrared filter is arranged for absorbing radiation having a wavelength which is substantially greater than circa 0.7 µm, so that also radiation having a wavelength greater than circa 0.7 µm can be absorbed. In this way, possible hazardous energy of specific lamp types, for instance a Xenon lamp, that generate a significant amount of radiation in the region of the spectrum with a wavelength greater than 0.7 µm, can be reduced. The utility of the illumination unit thus increases commensurately. In an alternative embodiment, however, the infrared filter is not designed for absorbing radiation, for instance when using types of lamps that emit only a small amount of radiation having said wavelength.

The light source 2 is designed as an arc lamp, for instance a Xenon lamp. Consequently, a relatively large amount of light can be generated, which is advantageous in the use of a multiple number of connectors for light guides with associated separate light paths, but also for tuning the light to the light guide to be connected, since the range in light yield is relatively large. In an alternative embodiment, a different type of lamp can be used, for instance a halogen lamp.

As shown in Fig. 1, the light source 2 is included in a frame 2A, such that only displacement in a height direction H is possible. In this way, the lamp 2 can be replaced relatively simply, while yet a good positioning in the plane transverse to the height direction can be realized. A good positioning in the plane transverse to the height direction is important to couple the generated light into the optical elements 4. For that matter, as is known to those skilled in the art, there are countless other conceivable constructions for including the light source 2.

The position of the light source 2 relative to the optical elements 4A, 4B is settable, in particular in the height direction H. In this way, the position of the light source 2, for instance after replacement, can be optimized relative to the optical elements in order to obtain optimum coupling of the generated light. The positioning of the light source 2 can for instance be realized using a stepper motor.

In addition, the illumination device has a sensor 14 for a position measurement of the position P where during operation the light is generated in the light source 2. Fig. 6 shows the light source 2 in more detail. Since an arc lamp is used, the position P where during operation the light is generated in the light source 2 is the location where the arc discharge between electrodes 16A, 16B takes place. The sensor 14 comprises for instance an array with photosensitive cells 15 which constitute a line detector extending in the height direction H. Furthermore, the illuminator 1 is provided with a feedback control for setting the position of the light source 2 on the basis of the measured position measurement. Thus, a displacement of the position P where during operation the light is generated in the light source 2, for instance due to progressive burning of an electrode of the arc lamp, can be compensated, so that the generated amount of light continues to be utilized optimally. It is noted that the sensor 14 can comprise a different type of position-sensitive sensor, such as a 4Q cell, a PSD or a duo diode. Furthermore, it is noted that an illumination unit 1 according to the invention can also be constructed without use of the sensor 14 or without the feedback, for instance if the location where the light is generated in the light source 2 exhibits no change or virtually no change during the period of operation of the light source 2 or if the amount of available light is amply sufficient.

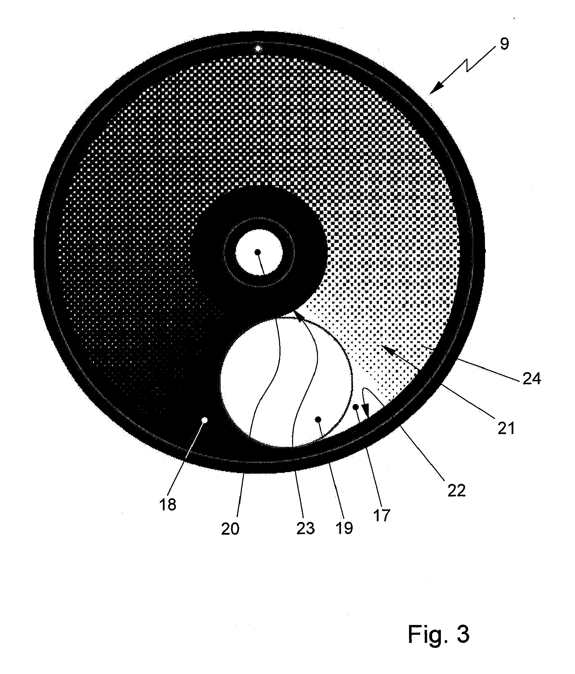

Each of the intensity filters 9A, 9B comprises a displaceable, substantially optically transparent carrier 17 which is provided with a substantially optically impermeable layer 18 with a coverage varying as a function of the location, as shown in Fig. 3. The displaceable, substantially optically transparent carrier 17 is designed as a swiveling disc 17. The disc 17 is manufactured from a plastic. Naturally, the disc 17 may also be manufactured from other materials, for instance glass. Furthermore, the disc 17 is provided with an opening 19 allowing locally unhindered passage of light exiting from the light source 2.

The intensity filter 9 is arranged in the illumination unit 1 so as to be rotatable about a central midpoint 20, such that in each position of the disc 17 an area from a ring-shaped segment 21 of the disc 17 is in the optical light path L. The ring-shaped segment 21 is bounded by an outer radius 22 and an inner radius 23 relative to the central midpoint 20 of the filter 9. An area that is in the optical light path L has such dimensions that it fits into the opening 19.

The optically impermeable layer 18 is formed by absorbing material that has been applied to the substantially optically transparent disc 17, so that incident light is partly absorbed. The coverage of the optically impermeable layer is a measure for the percentage of light that is allowed to pass. The coverage is locally substantially uniform and increases in a counterclockwise direction, so that by swiveling the filter 9 in a swiveling direction the intensity increases or decreases uniformly, that is, without intermediate respective decrease or increase. A locally substantially uniform coverage has been obtained by applying the material in a more or less balanced manner as relatively small or large areas 24 on the disc 17, so that a kind of matrix structure is formed. In this way, an even attenuation of the light beam can be realized, whereby the numerical aperture (NA) is not, or virtually not, affected.

Fig. 4 shows a schematic side elevation of an end of a light guide 25 connectible to the eye surgical illumination unit 1. The light guide 25 has a fastening element 26 for connection to a connector 3A, 3B of the illumination unit 1. The fastening element 26 is of tapered design, so that a proper alignment can be obtained. Naturally, the light guide 25 may also be aligned in a different manner. Furthermore, the fastening element 26 has been formed on an end 27 of the light guide 25 that has been stripped of a cladding 28. This end 27 of the light guide 25 is provided with an optical coding, implemented as a barcode with alternate light and dark strips.

After introducing the end 27 into a connector 3A, 3B of the illumination unit 1, identification of the light guide 25 is done by means of an optical measurement. The optical measurement can be performed with readily obtainable scan equipment. Furthermore, on the basis of information stored in a memory element of the illumination unit, such as setting characteristics of the light guide 25, information is generated for direct tuning of light exiting from the light source. This generated information contains for instance data about a maximum light yield of the light guide, such as an ISO value and/or an illumination value (lumen value). Such information is shown via a display panel 34 on the housing of the illumination unit. Thus, a user can observe directly, without use of additional documentation, what the optical performance of the connected light guide is and determine how long and with what intensity the light guide can be employed in a specific eye surgical treatment.

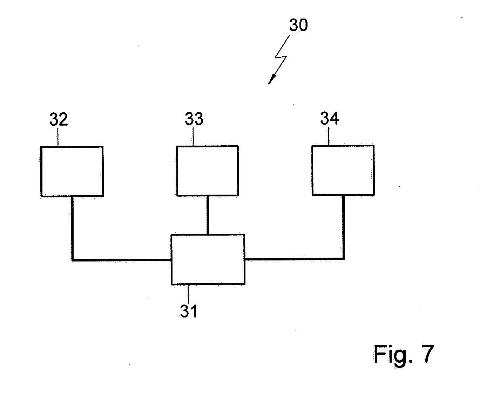

Various operations, such as executing the identification and generating information for direct tuning of the amount of light exiting from the illumination unit to an identified light guide, can optionally be carried out by dedicated program loaded into a computer system 30, comprising a processor 31 which is connected via data links to an optical measuring device 32 for performing the optical measurement, a memory element 33 for storing setting characteristics of light guides, and a display panel 34 for displaying the generated information of an identified light guide. Fig. 7 shows such a computer system.

In a special setting of the illumination unit 1, the intensity of the light exiting from the unit is automatically tuned to the identified light guide, based on the generated information on the light guide. Thus, the use of the illumination unit 1 is simplified still further and its utility is augmented still further. The user of the unit can set the spectral character of the exiting light with the aid of spectral filter settings. Furthermore, the user can determine the maximum duration for the use of the light source on the basis of the generated information on the light guide, especially on the basis of the so-called ISO values. The calculated ISO values can depend on the type of connected light guide, the intensity of the light that is coupled into the light guide, and spectral fiber settings. As described hereinabove, the intensity of the coupled light depends on the setting of the intensity filter. Furthermore, the illumination unit 1 may be so arranged that, notwithstanding manual setting possibilities, the amount of exiting light is actively limited to a maximum value at which the identified light guide can still be used without sustaining permanent damage. This prevents possible injury to the eye of a patient and damage to the light guide as a result of too high a light intensity.

The invention is not limited to the exemplary embodiments described here. Many variants are possible.

Thus, the illumination unit may be arranged for displaying the number of operating hours of the light source, so that the light source can be timely replaced so as to prevent untimely failure. Optionally, the number of operating hours displayed may be reset after replacement of the lamp. Also, the illumination unit may be arranged for displaying the number of operating hours still to be traversed until a pre-set replacement period has been completed.

Furthermore, identification of the light guide, instead of taking place in the illumination unit proper, can take place in a separate unit which is connected to the illumination unit via a data communication link.

In addition, when using a multiple number of connectors with associated separate light paths, a first connector may be arranged for connection to a first type of light guide and a second connector for connection to a second type of light guide, so that different types of light guides, for instance with different types of fastening elements, can be connected to the illumination unit.

Such variants will be clear to those skilled in the art and are understood to fall within the scope of the invention, as set forth in the following claims.

## Claims

1. An eye surgical illumination unit, comprising a light source for visual inspection of eye tissue to be treated, a connector for light guides for guiding light exiting from the light source to the eye tissue, and optical elements for defining a light path along which light exiting from the light source can travel to the connector, and a housing in which the light source and the optical elements are accommodated, wherein the illumination unit is arranged for an improved tuning of light operatively traveling along the light path, to a light guide connectible to the connector.

2. An eye surgical illumination unit according to claim 1, wherein the illumination unit is arranged for generating information based on a measurement on a light guide connectible to the connector, for direct tuning of light operatively exiting from the illumination unit via the connector.

3. An eye surgical illumination unit according to claim 1 or 2, comprising an optical measuring device for identifying a light guide connectible to the connector.

4. An eye surgical illumination unit according to claim 2 or 3, wherein the unit comprises a memory element for storing setting characteristics of light guides connectible to the illumination unit, and wherein the illumination unit is furthermore arranged for displaying setting characteristics of an identified light guide.

5. An eye surgical illumination unit according to claim 2 or 3, wherein the illumination unit is arranged for, in response to a measurement on a light guide connectible to the connector, tuning light operatively exiting from the illumination unit via the connector.

6. An eye surgical illumination unit according to any one of the preceding claims, wherein the optical elements comprise an intensity filter which comprises a displaceable, substantially optically transparent carrier which is provided with a substantially optically impermeable layer with a coverage varying as a function of the location.

7. An eye surgical illumination unit according to claim 6, wherein the displaceable, substantially optically transparent carrier is designed as a swiveling disc.

8. An eye surgical illumination unit according to claim 6 or 7, wherein the displaceable, substantially optically transparent carrier is provided with an opening for allowing locally unhindered passage of light exiting from the light source.

9. An eye surgical illumination unit according to any one of the preceding claims, wherein the light source is designed as an arc lamp.

10. An eye surgical illumination unit according to any one of the preceding claims, wherein the position of the light source relative to the optical elements is settable.

11. An eye surgical illumination unit according to claim 10, furthermore comprising a sensor for a position measurement of the position in the light source where during use of the unit the light is generated and a feedback control for setting the position of the light source based on the position measurement.

12. An eye surgical illumination unit according to any one of the preceding claims, wherein the numerical aperture of the optical elements is substantially greater than circa 0.5.

13. An eye surgical illumination unit according to any one of the preceding claims, wherein the optical elements comprise a reflector for reflecting light exiting from the light source, wherein a reflector surface of the reflector is provided with an infrared filter.

14. An eye surgical illumination unit according to claim 13, wherein the infrared filter is arranged for absorbing radiation having a wavelength that is substantially greater than circa 0.7 µm.

15. An eye surgical illumination unit according to any one of the preceding claims, wherein the optical elements comprise a spherical reflector for reflecting light exiting from the light source, wherein the geometric center of the reflector is slightly offset relative to the position in the light source where during use of the unit the light is generated.

16. An eye surgical illumination unit according to any one of the preceding claims, further comprising an additional connector for light guides and additional optical elements for defining a separate light path along which light exiting from the light source can travel to the additional connector.

17. A light guide for eye surgical applications, comprising a fastening element for connection to a connector of an eye surgical illumination unit, further comprising an optical identification code.

18. A method for readying for use an eye surgical illumination unit to which a light guide is connectible, wherein an improved tuning of light operatively exiting from the illumination unit to the connected light guide is realized.

19. A method according to claim 18, comprising generating information for direct tuning of light operatively exiting from the light source, based on a measurement on a light guide connectible to the illumination unit.

20. A method according to claim 18 or 19, comprising, in response to a measurement on a light guide connectible to the illumination unit, tuning light operatively exiting from the illumination unit.

21. A computer program product comprising instructions for having a processor carry out the method for readying for use an eye surgical illumination unit to which a light guide is connectible, comprising identifying the light guide with the aid of an optical measurement.

22. A computer program product according to claim 21, furthermore comprising instructions for having a processor carry out the method of tuning light operatively exiting from the illumination unit on the basis of setting characteristics of the identified light guide.

23. A computer system, comprising a processor for carrying out a method for readying for use an eye surgical illumination unit to which a light guide is connectible, further comprising an optical measuring device which is connected to the processor with a data link.
